# EUROPÄISCHE PATENTSCHRIFT

(11) **EP 1 867 303 B1**
(45) Veröffentlichungstag und Bekanntmachung des Hinweises auf die Patenterteilung: **01.09.2010**
(21) Anmeldenummer: 07004303.9
(22) Anmeldetag: 02.03.2007
(51) Int. Cl.: A61F 2/40

(54) **Oberflächenersatzprothese für den Humeruskopf**
Surface replacement prosthesis for the humerus head
Prothèse de surface pour tête d'humérus

(30) Priorität: 16.06.2006 DE 102006028136
(43) Veröffentlichungstag der Anmeldung: 19.12.2007
(73) Patentinhaber: implantcast GmbH, 21614 Buxtehude (DE)
(72) Erfinder: Jerosch, Jörg, Prof. Dr. med., 41472 Neuss (DE)
(74) Vertreter: Raffay & Fleck

(56) Entgegenhaltungen:
- EP-A- 1 518 519
- EP-A- 1 570 816
- EP-A1- 0 485 152
- DE-A1- 3 917 285
- US-A- 5 944 757

## Beschreibung

Die Erfindung betrifft eine Schultergelenksprothese, genauer eine Oberflächenersatzprothese für den Humeruskopf mit den Merkmalen des Oberbegriffs des Patentanspruchs 1.

Durch die Dezentrierung des Humeruskopfes bei Patienten mit einer Rotatorenmanschettendefektarthropathie kommt es zu einem massiven Funktionsverlust des Schultergelenkes. Neben dem Funktionsverlust entstehen dem Patienten auch erhebliche Schmerzen. Dieses Problem war über viele Jahrzehnte hin nicht lösbar.

Um hier Abhilfe zu schaffen, wurden verschiedene Überlegungen zu Prothesen für das Schultergelenk angestellt. Eine Möglichkeit wurde mit sogenannten inversen Schulterprothesen verfolgt. Hierbei wird an dem ursprünglich den kugeligen Gelenkkopf ausbildenden Humeruskopf ein Prothesenelement mit einer konkaven Gelenkpfanne implantiert, ein entsprechend kugelig konvex geformtes Gegenstück wird anstelle der ursprünglichen Gelenkpfanne im Schulterknochen verankert. Beschrieben ist diese Technik z.B. in dem Artikel "Grammont inverted total shoulder arthroplasty in the treatment of glenohumeral osteoarthritis with massive rupture of the cuff", THE JOURNAL OF BONE AND JOINT SURGERY 2004, Vol. 86, Seiten 388 ff. Der Vorteil dieser Methode liegt in der Distalisierung des Humeruskopfes, also seiner Verlagerung vom Körperzentrum weg. Dadurch kann der Musculus deltoideus wieder eine Abduktion des Armes im Schultergelenk bewirken, die Funktionalität des Gelenkes ist wiederhergestellt. Nachteilig bei der inversen Prothese ist jedoch u.a., dass es zu erheblichen Kräften auf die glenoidale Verankerung kommt und dass die humerale Komponente bei einer Abduktion des Armes an der Skapula (dem Schulterblatt) anstößt. Dieses Phänomen führt vielfach zu Lockerungen des Implantates. Zudem macht das Einsetzen einer inversen Schulterprothese einen erheblichen Eingriff an der Schulter erforderlich mit einem hohen Knochenverlust. Dies führt letztlich dazu, dass es bei einer erneuten Funktionsstörung des Gelenkes nur noch sehr wenige operative Rückzugsmöglichkeiten für den erneuten oder veränderten Einsatz einer Prothese gibt, so dass dann oftmals nur noch eine dauerhafte Entfernung des Schultergelenkes möglich ist.

Um den großen Knochenverlust bei der Implantation der inversen Prothese zu vermeiden und um noch operative Rückzugsmöglichkeiten im Falle einer Revision zu haben, werden seit einiger Zeit sogenannte Oberflächenprothesen bei Patienten mit Defektarthropathie implantiert. Diese Oberflächenprothesen werden auf den beschädigten Humeruskopf aufgesetzt, der zuvor durch Knochenabtrag entsprechend vorgeformt wurde. Bei dieser Implantationsmethode wird folglich ein minimaler Knochenverlust generiert, der zudem nur den Humeruskopf, nicht aber die weiteren Knochen des Schultergelenkes betrifft. Beschrieben ist eine solche Oberflächenprothese z.B. in dem Artikel Der endoprothetische Oberflächenerstaz am Humeruskopf" in der Zeitschrift der Orthopäde Nr. 6/2001 Seiten 79 ff. Die bekannten Oberflächenprothesen für den Humeruskopf setzen sich zusammen aus einer runden Kappe mit im wesentlichen durchgehend gleicher Materialstärke und einem im Zentrum der Kappe in derem Inneren angesetzten Befestigungsdorn bzw. -zapfen.

Die bekannten Oberflächenprothesen haben sich bewährt, insbesondere bei Patienten, die bei einer Defektarthopathie noch unter 70 Jahren alt sind, da diese grundsätzlich möglicherweise für eine Revision der Prothese in Betracht zu ziehen sind. Bei den bekannten Oberflächenprothesen wird allerdings nicht der bei der inversen Prothese als vorteilhaft erkannte Effekt einer Distalisierung des Humeruskopfes erzielt. Vielmehr behält der Humeruskopf seine ursprüngliche Position, was je nach Erkrankungsbild insbesondere für die Funktion des Musculus deltoideus und damit einer Abduktion des Armes von Nachteil sein kann.

In der EP 1 518 519 A2 ist eine Oberflächenersatzprothese für den Humeruskopf mit allen Merkmalen des Oberbegriffs des Anspruchs 1 beschrieben. Durch die im Zentrum der Prothese größere Wandstärke als in den äußeren Bereichen wird eine Distalisierung des Humeruskopfes erreicht, wie sie bei der inversen Prothese erzielt werden kann, ohne dass es bei der Implantation der Prothese zu einem wie bei den inversen Prothesen erforderlichen Knochenverlust kommt. Dieser Vorteil soll bei einer weiterzubildenden Oberflächenersatzprothese beibehalten werden, die also auch die Vorteile der beiden eingangs genannten-Prothesensysteme miteinander vereinen soll.

Ein Problem bei der Verwendung einer solchen Oberflächenersatzprothese besteht darin, dass die Subskapularissehne wieder am neu geschaffenen System befestigt werden muss. Dies muss sicher und möglichst dauerhaft geschehen.

So soll also die gegenüber dem Stand der Technik weiterzubildende Oberflächenersatzprothese auch die Möglichkeit schaffen, die Nähte für die Refixation der Subskapularissehne durch den Knochen zu führen, ohne dass etwa die Gefahr besteht, dass diese Nähte bei zyklischer Belastung eine Beschädigung erfahren.

Erfindungsgemäß wird diese Aufgabe gelöst durch eine Oberflächenersatzprothese mit den Merkmalen des Anspruches 1.

Zur Lösung dieser Aufgabe weist erfindungsgemäß der innere Rand entlang der Innenkante der Kappe im Übergangsbereich von Oberseite zu der Unterseite eine besonders glättend behandelte, insbesondere hochfein polierte, Oberfläche auf. Auf diese Weise ist es möglich, die Nähte für die Refixation der Subskapularissehne durch den Knochen zu führen, ohne dass die Gefahr besteht, dass diese Nähte bei zyklischer Belastung durch den Prothesenrand zerrieben werden. Ohne die in dieser Weiterbildung angegebene besonders glättend behandelte, hochfein polierte Oberfläche bestünde die Gefahr, dass Rauhigkeiten auf der Oberfläche diese Nähte durch Abrieb beschädigen und letztlich zerstören.

Ferner weist nach der Erfindung die Kappe im Randbereich eine geringere Materialstärke auf als in der Mitte, wobei die Materialstärke in der Mitte wegen des dort angesetzten Verankerungsdornes bzw. -zapfens gemessen wird von einem weiter geführten, gedachten Verlauf der Unterseite bis zur Oberseite der Kappe.

Durch diese Maßnahme wird der Abstand zwischen dem Akromion und dem Rotationszentrum des Humeruskopfes vergrößert, wodurch sich hinsichtlich der Distalisierung des Humeruskopfes vergleichbare Effekte ergeben wie bei der Implantation einer inversen Prothese. Zugleich bleiben jedoch die erheblichen Vorteile des deutlich geringeren Knochenverlustes bei der Implantation der erfindungsgemäßen Oberflächenprothese im Vergleich zur Implantation einer inversen Prothese. Dies führt letztlich auch zu einer deutlich verkürzten Operationszeit, einem geringeren Blutverlust und einer schnelleren Rehabilitation des Patienten. Darüber hinaus erlaubt die erfindungsgemäße Oberflächenprothese im Falle einer notwendigen Revision noch weitere Rückzugsoptionen (z.B. den Wechsel auf andere Schaftprothesen, den Wechsel auf eine inverse Prothese oder dgl.). Bei der Operation bietet sich zudem an, die erfindungsgemäße Oberflächenprothese etwas valgisch zu implantieren, so dass das Implantat mit dem Akromion und dem coracoacromialen Bogen artikuliert. Klinisch zeigt sich hierdurch eine weitere Schmerzreduktion des Patienten.

Die variable Wandstärke der Kappe wird z.B. erreicht durch unterschiedliche Durchmesser einer sphärisch ausgebildeten Oberseite und einer entsprechenden Unterseite oder aber durch versetzte Mittelpunkte. Selbstverständlich kann die unterschiedliche Stärke der Kappe durch sämtliche geeigneten Maßnahmen erhalten werden.

Mit Vorteil ist die Kappe so gestaltet, dass ihre Dicke sich ausgehend von der Mitte (dem Polbereich) hin zu den Randbereichen kontinuierlich verjüngt (Anspruch 2).

Als vorteilhafte Stärken bzw. Dicken der Kappe haben sich im Polbereich Wandstärken zwischen 8 mm und 18 mm, vorzugsweise zwischen 11 mm und 15 mm und am Rand zwischen 2 mm und 6 mm, vorzugsweise zwischen 2 mm und 4 mm, herausgestellt, wobei die Stärke im Polbereich (in der Mitte) zwischen der Oberseite und dem im Anspruch 1 bzw. im Anspruch 3 angegebenen Punkt auf einer gedachten Fortführung der Kontur der Unterseite gemessen ist. Mit diesen Abmessungen konnten hinsichtlich der Motorik des Schultergelenkes nach der Implantation die besten Ergebnisse erzielt werden.

Der Verankerungsdorn bzw. -zapfen ist bevorzugt als in seinem Inneren hohler Einschlagdorn ausgebildet mit seitlich daran angeordneten, in seiner Längsrichtung verlaufenden Verankerungsfinnen. Dabei kann der Verankerungsdorn bzw.
- zapfen sich zu seinem freien Ende hin konisch verjüngend ausgebildet sein. Die Ausbildung des Verankerungsdornes bzw.
- zapfens in dieser Weise eignet sich besonders für eine zementfreie Verankerung der Oberflächenersatzprothese, wobei die Verankerungsfinnen einen rotationsfesten Sitz einer Oberflächenersatzprothese sicherstellen.

Mit Vorteil ist die Oberflächenersatzprothese, zumindest die Oberseite der Kappe, mit einer keramischen TiN-Beschichtung überzogen. Diese Beschichtung dient einer Verbesserung der tribologischen Eigenschaften und ermöglicht die Versorgung nickelallergischer Patienten mit der erfindungsgemäßen Prothese bei gleichzeitiger Verbesserung der Abriebeigenschaften.

Weitere Vorteile und Merkmale der Erfindung ergeben sich aus der nachfolgenden Beschreibung eines Ausführungsbeispiels anhand der beigefügten Figuren. Dabei zeigen:
- Fig. 1: eine dreidimensionale Ansicht eines Ausführungsbeispiels für eine erfindungsgemäße Oberflächenersatzprothese;
- Fig. 2: eine Seitenansicht der Oberflächenersatzprothese aus Fig. 1;
- Fig. 3: eine Schnittansicht der Oberflächenersatzprothese aus Fig. 2 entlang der dort aufgezeigten Schnittlinie A - A;
- Fig. 4: eine schematische Darstellung eines natürlichen Schultergelenkes;
- Fig. 5: eine schematische Darstellung eines Schultergelenkes mit einer implantierten Oberflächenersatzprothese nach dem Stand der Technik;
- Fig. 6: eine schematische Darstellung eines Schultergelenkes mit einer inversen Prothese; und
- Fig. 7: eine schematische Darstellung eines Schultergelenkes mit einer erfindungsgemäßen Oberflächenersatzprothese.

In den Figuren sind gleiche Elemente mit gleichen Bezugszeichen versehen.

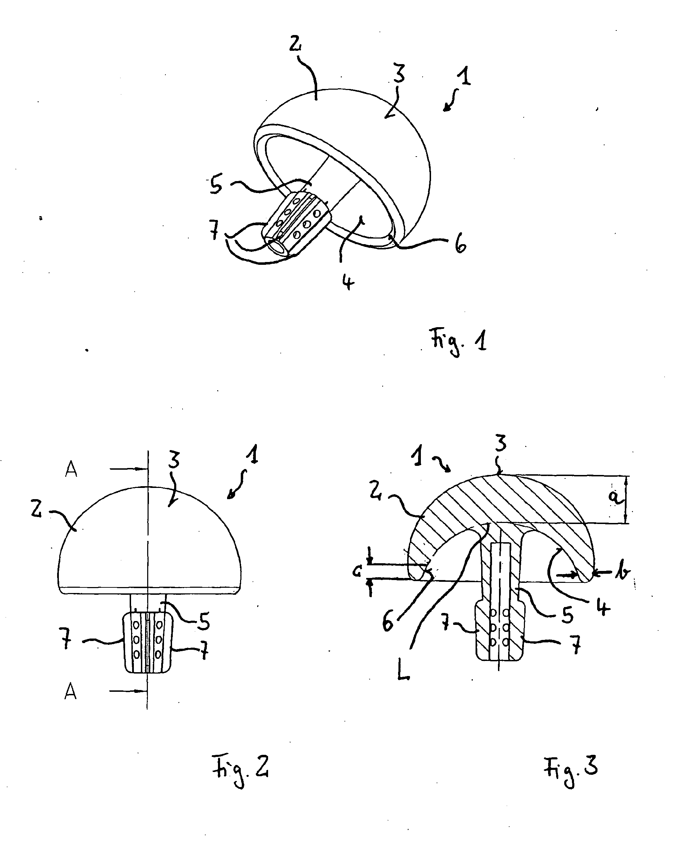

In den Figuren 1 bis 3 ist zunächst in verschiedenen Ansichten eine erfindungsgemäße Oberflächenersatzprothese dargestellt und allgemein mit 1 bezeichnet. Die Oberflächenersatzprothese 1 ist einstückig ausgebildet aus TiAl₆V₄ und ist mit einer keramischen TiN-Beschichtung überzogen. Sie setzt sich zusammen aus einer Kappe 2 mit einer sphärisch geformten, konvex gekrümmten Oberseite 3 und einer ebenfalls sphärisch geformten, jedoch konkav gekrümmten Unterseite 4 und einem einstückig an der Kappe 2 an deren Unterseite 4 angeformten Verankerungsdorn 5. Insgesamt hat die Oberflächenersatzprothese 1 ein pilz- bzw. champignonförmiges Aussehen.

Der innere Rand 6 der Kappe 2 ist hoch poliert ausgebildet mit einer besonders glatten und ebenen Oberfläche.

Der Verankerungsdorn 5 ist in seinem Innern hohl ausgebildet und verläuft zu seinem freien Ende sich konisch verjüngend mit kreisförmigem Durchmesser. An seinem freien Ende sind Verankerungsfinnen 7 angeordnet, die sich entlang der Längsrichtung des Verankerungsdorns 5 erstrecken und radial von diesem abstehen.

Insbesondere in Fig. 3 ist gut zu erkennen, dass die Kappe in einem mittleren Bereich (Polbereich) dicker mit größerer Materialstärke ausgebildet ist als am Rand. Veranschaulicht wird dies durch den Abstand a zwischen der Oberseite 3 und einer die Kontur der Unterscheite 4 fortführenden, gedachten Linie L im mittleren Bereich der Kappe 2, der deutlich größer ist als der Abstand b zwischen der Oberseite 3 und der Unterseite 4 der Kappe 2 im Randbereich. In der Praxis beträgt der Abstand a in diesem Ausführungsbeispiel 13 mm, der Abstand b etwa 3 bis 4 mm.

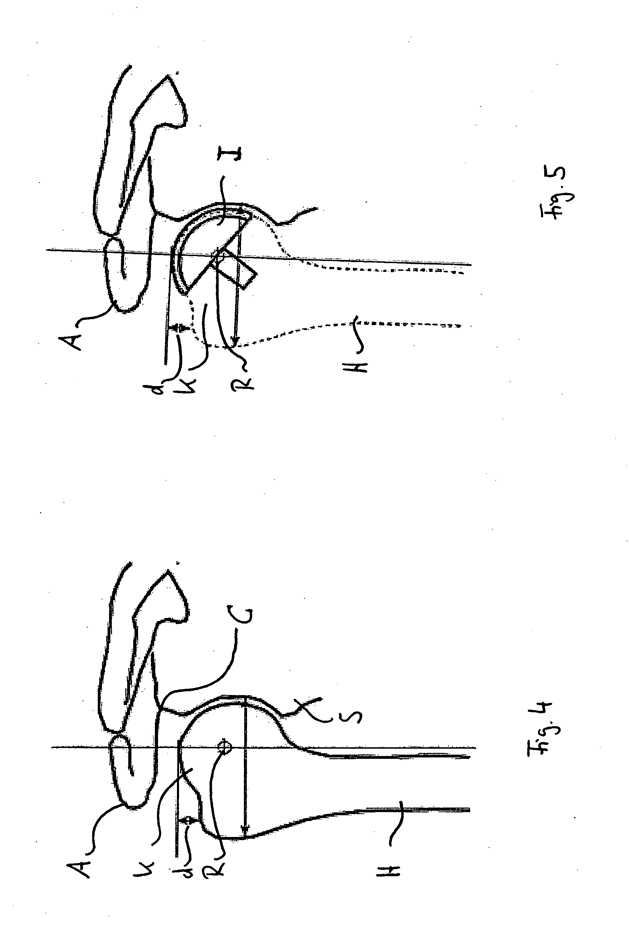

Die durch diese erfindungsgemäße Ausgestaltung der Oberflächenersatzprothese 1 zu erzielenden Vorteile werden nachfolgend anhand der Darstellungen der Figuren 4 bis 7 eingehender beschrieben. Zuvor soll allerdings noch auf die Ausgestaltung des inneren Randes 6 sowie des Verankerungsdornes 5 eingegangen werden.

Der oben geschilderte Aufbau des Verankerungsdorns 5 mit dem Hohlraum im Inneren und den Verankerungsfinnen 7 ermöglicht eine zementfreie Verankerung der Oberflächenersatzprothese 1 durch Einschlagen in den Humeruskopf. Die Verankerungsfinnen 7 sorgen für eine drehsicherere Befestigung und verhindern so ein Ablösen der Oberflächenersatzprothese 1 vom Humerus durch Herausdrehen.

Der bis zum Abstand c, welcher in diesem Ausführungsbeispiel bevorzugt 3 bis 4 mm beträgt, hochglatt polierte innere Rand 6 erlaubt es, die Nähte für die Refixation der Subskapularissehne durch den Knochen zu führen, ohne dass die Gefahr besteht, dass diese Nähte bei zyklischer Belastung durch eventuelle Rauhigkeiten an dem inneren Rand 6 zerrieben werden und sich die Subskapularissehne so ablöst.

Nachfolgend soll nun die Wirkweise der erfindungsgemäßen Oberflächenersatzprothese 1 anhand der verschiedenen Darstellungen in den Figuren 4 bis 7 erläutert werden.

In Fig. 4 ist zunächst ein natürliches Schultergelenk schematisch dargestellt. Gezeigt ist hier der Oberarmknochen (Humerus) H mit seinem Humeruskopf K, wie er in der Gelenkpfanne des Schulterblattes S ruht. Weiterhin gezeigt sind die das Gelenk begrenzenden Knochen bzw. Elemente Akromion A und coracoacromialer Bogen C. Mit R schließlich ist das Rotationszentrum des Humeruskopfes H bezeichnet.

In Fig. 5 ist ein mit einer herkömmlichen Oberflächenersatzprothese I versehener Humerus H bzw. Humeruskopf K in einem ansonsten unverändert belassenen Schultergelenk gezeigt. Der Vergleich mit Fig. 4 zeigt, dass das Rotationszentrum R sich nicht verändert hat und ebenso wenig der Abstand zwischen dem Humeruskopf K und dem Akromion bzw. die diesen repräsentierende Distalisierung d.

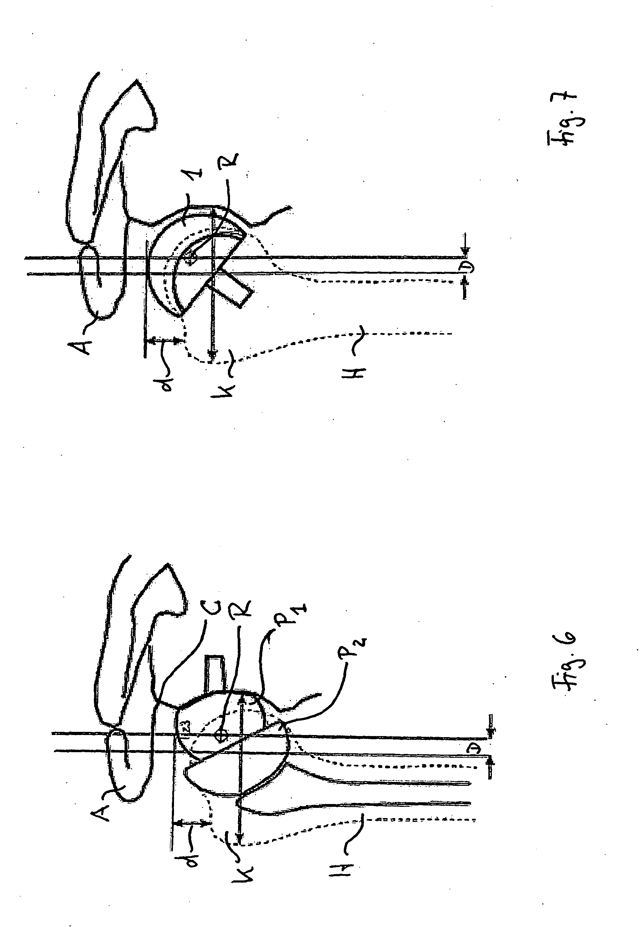

In Fig. 6 ist die Situation bei einer aus einem im Schulterblatt verankerten Kugelteil P₁ und einem im Humerus angebrachten Pfannenteil P₂ gebildeten inversen Implantat dargestellt. Diese Art der Prothese führt zu einer deutlichen Absenkung, Distalisierung, des Humerus gegenüber der natürlichen Situation, was sich in einem größeren Abstand d äußert. Ferner wird bei dieser Prothese eine Medialisierung des Rotationszentrums R erzielt, was sich durch den in der Figur gezeigten und als Lateralisierung bezeichneten Abstand D ausdrückt. In der Figur kann auch erkannt werden, dass der Verlust an Knochenmaterial bei der Anbringung dieser inversen Prothese deutlich größer ist, als bei der Implantation der herkömmlichen Oberflächenersatzprothese I aus Fig. 2.

In Fig. 7 ist schließlich eine Situation dargestellt, in welcher eine erfindungsgemäße Oberflächenersatzprothese 1 in den Humeruskopf K implantiert ist. Deutlich ist auch hier zu erkennen, dass sich eine Distalisierung d ergibt, die mit derjenigen der in Fig. 6 dargestellten inversen Prothese vergleichbar ist, und dass das Rotationszentrum R im Humeruskopf K in Richtung der Körpermitte gewandert ist, was sich in der Lateralisierung D ausdrückt. Beide Effekte lassen sich zurückführen auf die im mittleren Bereich überhöht ausgebildete Kappe 2 der Oberflächenersatzprothese 1. Diese schafft die in der Fig. 7 dargestellten Position erkennbare Verlagerung des Humerus mit der positiven Folge, dass der Musculus deltoideus wieder eine Abduktion des Armes bewirken kann.

Aus der obigen Beschreibung eines Ausführungsbeispieles und der vergleichenden Darstellung der verschiedenen Implantate sind die Vorteile und Merkmale der Erfindung für einen Fachmann noch einmal deutlich geworden. Dabei soll hier noch einmal betont werden, dass das Ausführungsbeispiel die Erfindung in ihrem Umfang nicht beschränkt, sondern lediglich der Erläuterung und dem besseren Verständnis dient. Der Umfang der Erfindung wird in vollem Ausmaß durch die nachfolgenden Patentansprüche definiert.

### Bezugszeichenliste

- 1: Oberflächenersatzprothese
- 2: Kappe
- 3: Oberseite
- 4: Unterseite
- 5: Verankerungsdorn
- 6: innerer Rand
- 7: Verankerungsfinnen

- a: Abstand
- b: Abstand
- c: Abstand
- d: Distalisierung

- A: Akromion
- C: coracoacromialer Bogen
- D: Lateralisierung
- H: Humerus
- I: Oberflächenersatzprothese
- K: Humeruskopf
- L: Linie
- P₁: Kugelteil
- P₂: Pfannenteil
- S: Schulterblatt
- R: Rotationszentrum

## Patentansprüche

1. Oberflächenersatzprothese für den Humeruskopf (K) mit einer Kappe (2), welche eine die Gelenkoberfläche bildende, gekrümmte Oberseite (3) und eine zur Auflage auf dem operativ bearbeiteten Humeruskopf (K) vorgesehenen gekrümmte Unterseite (4) aufweist, und mit einem an der Unterseite (4) festgelegten Verankerungsdorn bzw. -zapfen (5) zur Verankerung im Oberarmknochen (H), bei welcher der Abstand (b) zwischen der Oberseite (3) und der Unterseite (4) in den Randbereichen der Kappe (2) kleiner ist als der Abstand (a) zwischen der Oberseite (3) und einem auf einer kontinuierlichen Weiterführung der Konturlinie (L) der Unterseite (4) in den Seitenbereiche der Oberseite (3) radial gegenüberliegenden Punkt in der Mitte, entsprechend dem Polbereich, der Kappe (2), **dadurch gekennzeichnet, dass** die Kappe (2) im Übergangsbereich von Oberseite (3) zu der Unterseite (4) an ihrem inneren Rand (6) eine besonders glättend behandelte, insbesondere hoch fein polierte, Oberfläche aufweist.

2. Oberflächenersatzprothese nach Anspruch 1, **dadurch gekennzeichnet, dass** die Kappe (2) sich ausgehend von der Mitte hin zu den Randbereichen kontinuierlich verjüngend ausgebildet ist.

3. Oberflächenersatzprothese nach einem der vorhergehenden Ansprüche, **dadurch gekennzeichnet, dass** der Abstand (a) zwischen der Oberseite (3) und dem auf einer kontinuierlichen Weiterführung der Konturlinie (L) der Unterseite (4) radial gegenüberliegende Punkt zwischen 8 mm und 18 mm, vorzugsweise zwischen 11 mm und 15 mm, beträgt und dass der Abstand (b) zwischen der Oberseite (3) und der Unterseite (4) am Rand der Kappe (2) zwischen 2 mm und 6 mm, vorzugsweise zwischen 2 mm und 4 mm, beträgt.

4. Oberflächenersatzprothese nach einem der vorhergehenden Ansprüche, **dadurch gekennzeichnet, dass** der Verankerungsdorn bzw. -zapfen (5) als in seinem Inneren hohler Einschlagdorn ausgebildet ist mit seitlich angeordneten, in seiner Längsrichtung verlaufenden Verankerungsfinnen (7).

5. Oberflächenersatzprothese nach einem der vorhergehenden Ansprüchen, **dadurch gekennzeichnet, dass** sie, zumindest die Oberseite der Kappe (2), mit einer keramischen TiN-Beschichtung überzogen ist.

## Claims

1. A replacement and resurfacing prosthesis for humerus head (K) fitted with a cap (2), having a bent upper face (3), forming the surface articulation and a lower face (4) provided for assembly on the operational humerus head (K), and an anchor bolt or lug (5) secured to the lower face (4) for anchoring into the bone of the upper arm (H), wherein between the distance (b) between the upper face (3) and the lower face (4) in the edges of the cap (2) is smaller than the distance (a) between the upper face (3) and a radial point opposite the centre, over a continuous extension of the contour line (L) of the lower face (4) in the lateral sides of the lower face (3) corresponding to the polar are of the cap (2), **characterised in that** the cap (2) exhibits in the transition area from the upper face (3) to the lower face (6), at the lower edge thereof, an extremely polished, smoothened surface

2. A replacement and resurfacing prosthesis according to claim 1, **characterised in that** the cap (2) is continuously tapering from the centre towards the edges.

3. A replacement and resurfacing prosthesis according to any of the previous claims, **characterised in that** the cap (a) ranges between 8 mm and 18 mm, preferably between 11 mm and 15 mm, between the upper face (3) and the opposite radial point on the continuous extension of the contour line (L) of the lower face (4) and that the distance (b) ranges between 2 mm and 6 mm, preferably between 2 mm and 4 mm, between the upper face (3) and the lower face (4) at the edge of the cap.

4. A replacement and resurfacing prosthesis according to any of the previous claims, **characterised in that** the anchoring bolt or lug (5) is in the form of a hollow tapping punch in its inner portion with anchor fins (7) arranged laterally in the longitudinal axis.

5. A replacement and resurfacing prosthesis according to any of the previous claims, **characterised in that** it is covered with a ceramic TiN coating, at least the upper face of the cap (2).

## Revendications

1. Prothèse de remplacement et de resurfaçage pour tête humérale (K) munie d'un capuchon (2), présentant une face supérieure (3) recourbée, formant l'articulation de surface et une face inférieure (4) prévue pour installation sur la tête humérale (K) opérationnelle,et un boulon ou tenon d'ancrage (5) fixé sur la face inférieure (4) pour ancrage dans l'os du bras supérieur (H), a distance (b) entre la face supérieure (3) et la face inférieure (4) dans les bords du capuchon (2) est plus petite que la distance (a) entre la face supérieure (3) et un point opposé radialement au centre, sur un prolongement continu de la ligne de contour (L) de la face inférieure (4) dans les parties latérales de la face supérieure (3), correspondant à la zone polaire du capuchon (2), **caractérisée en ce que** le capuchon (2) présente dans la zone de transition de la face supérieure (3) vers la face inférieure (6), au bord intérieur de celle-ci, une surface extrêmement polie, ayant subi en traitement de lissage

2. Prothèse de remplacement et de resurfaçage selon la revendication 1, **caractérisée en ce que** le capuchon (2) va rétrécissant de façon continue du centre vers les bords.

3. Prothèse de remplacement et de resurfaçage selon l'une quelconque des revendications précédentes, **caractérisée en ce que** le capuchon (a) est compris entre 8 mm et 18 mm, de préférence entre 11 mm et 15 mm, entre la face supérieure (3) et le point opposé radial sur le prolongement continu de la ligne de contour (L) de la face inférieure (4) et que la distance (b) est comprise entre 2 mm et 6 mm, de préférence entre 2 mm et 4 mm, entre la face supérieure (3) et la face inférieure (4) au bord du capuchon.

4. Prothèse de remplacement et de resurfaçage selon l'une quelconque des revendications précédentes, **caractérisée en ce que** le boulon ou tenon d'ancrage (5) prend la forme d'un pointeau de frappe creux dans sa partie interne avec des ailettes d'ancrage (7) disposées latéralement dans l'axe longitudinal..

5. Prothèse de remplacement et de resurfaçage selon l'une quelconque des revendications précédentes, **caractérisée en ce qu'**elle est enduite d'un revêtement TiN céramique, au moins la face supérieure du capuchon (2).
